# EUROPEAN PATENT APPLICATION

(11) **EP 4 272 671 A1**
(43) Date of publication of application: **08.11.2023**
(21) Application number: 21914572.9
(22) Date of filing: 29.12.2021
(51) Int. Cl.: A61B 18/12, A61B 34/00, A61B 18/14

(54) **RADIO-FREQUENCY OPERATION PROMPTING METHOD BASED ON MULTI-POLE RADIO-FREQUENCY ABLATION CATHETER, APPARATUS, SYSTEM, AND STORAGE MEDIUM**

(30) Priority: 31.12.2020 CN 202011637715
(71) Applicant: Hangzhou Broncus Medical Co., Ltd., Hangzhou, Zhejiang 310051 (CN)
(72) Inventor: XU, Hong, Hangzhou, Zhejiang 310051 (CN)
(74) Representative: Petraz, Gilberto Luigi
(86) International application number: PCT/CN2021/142751
(87) International publication number: WO 2022/143840

(57) **Abstract**

A radiofrequency operation prompting method based on a multi-pole radiofrequency ablation catheter, an apparatus, a system, and a storage medium are disclosed. The method includes: a computer terminal obtaining physical characteristic data of an operation location of a radiofrequency operation object in real time by means of probes in multiple side electrodes that are distributed on the multi-pole radiofrequency ablation catheter at intervals along the circumferential direction; obtaining a physical characteristic field of the radiofrequency operation object according to the data obtained in real time; and according to an initial range of a target operation area of the radiofrequency operation object and the value change of the physical characteristic data in the physical characteristic field, obtaining a range change of an to-be-operated area, and displaying the range change by means of a three-dimensional model. According to the present disclosure, a visual prompt of the range change of the to-be-operated area can be achieved, so that the success rate and effect of radiofrequency operation can be improved.

## Description

### TECHNICAL FIELD

The embodiments of the present disclosure relate to the technical field of data processing, and in particular to a radiofrequency operation prompting method based on a multi-pole radiofrequency ablation catheter, apparatus, system and storage medium.

### DESCRIPTION OF THE PRIOR ART

Radiofrequency technology refers to a technology where, under the guidance of an image, a radiofrequency probe enters the operation location of the radiofrequency operation object, and a radiofrequency host sends radiofrequency signals to the radiofrequency operation object to complete the radiofrequency operation. During the radiofrequency operation, the radiofrequency operation is important for guaranteeing the radiofrequency effect.

In the prior art, when the radiofrequency host performs radiofrequency operation, a simple form of digital is generally displayed on a display interface in to prompt the user the current operation state of the radiofrequency operation. However, this prompting method is not precise enough, the prompting information is simple, and doesn't work well, so the prompting effect is poor, which further affects the radiofrequency operation.

### SUMMARY OF THE DISCLOSURE

The radiofrequency operation prompting method based on the multi-pole radiofrequency ablation catheter, apparatus, system and storage medium according to the embodiments of the present disclosure can realize the visual prompt of the range change of the to-be-operated area, thereby improving the effectiveness of the information prompt and thus the success rate and effect of radiofrequency operation.

In one aspect, an embodiment of the present disclosure provides a radiofrequency operation prompting method based on a multi-pole radiofrequency ablation catheter, which is applied to a computer terminal, and the method includes:
Obtaining physical characteristic data of the operation location of the radiofrequency operation object in real time through a plurality of probes of the multi-pole radiofrequency ablation catheter;
Obtaining the physical characteristic field of the radiofrequency operation object according to the physical characteristic data obtained in real time;
Obtaining the range change of the to-be-operated area in the target operation area according to the initial range of the target operation area of the radiofrequency operation object and the value change of the physical characteristic data in the physical characteristic field, and representing the range change through a three-dimensional model;
The multi-pole radiofrequency ablation catheter includes a handle with a proximal end and a distal end, an outer catheter assembly with a proximal end and a distal end, and an inner catheter assembly with a proximal end and a distal end;
The proximal end of the outer catheter assembly is connected to the distal end of the handle;
The proximal end of the inner catheter assembly is connected to the distal end of the handle;

The inner catheter assembly includes a branched electrode assembly that can be driven by the handle to rotate relative to the outer catheter assembly, and the branched electrode assembly includes a plurality of side electrodes arranged at intervals in the circumferential direction, and the side electrodes include the probes.

In another aspect, an embodiment of the present disclosure further provides an electronic apparatus, including:
A memory and a processor;
The memory stores executable program codes;
The processor is coupled with the multi-pole radiofrequency ablation catheter and the memory and configured to invoke the executable program code stored in the memory to execute the radiofrequency operation prompting method based on the multi-pole radiofrequency ablation catheter according to the above embodiment.

In a further aspect, the embodiment of the present disclosure provides a non-transitory computer-readable storage medium, in which a computer program is stored. When the computer program is executed by a processor, the radiofrequency operation prompting method based on the multi-pole radiofrequency ablation catheter according to the above-mentioned embodiment is implemented.

In the embodiments in the present disclosure, by using a plurality of probes of the multi-pole radiofrequency ablation catheter, a plurality of physical characteristic data at the radiofrequency operation location are obtained in real time, and the physical characteristic field of the radiofrequency operation object is obtained according to these data, and then according to the initial range of the target operation area of the radiofrequency operation object and the value change of the physical characteristic data in the physical characteristic field, the range change of the to-be-operated area in the target operation area can be obtained and represented through the three-dimensional model, realizing a visual prompt of range change of the to-be-operated area with more abundant, intuitive and vivid prompt information, improving the accuracy and intelligence of determining the to-be-operated area, thereby improving the effectiveness of the information prompt and thus the success rate and effect of radiofrequency operation.

### BRIEF DESCRIPTION OF DRAWINGS

In order to more clearly illustrate the technical solutions of the embodiments of the present disclosure or the prior art, the drawings that need to be referred in the description of the embodiments or the prior art will be briefly introduced in the following. Obviously, the drawings in the following only show some embodiments of the present disclosure. For those skilled in the art, other drawings can also be obtained according to these drawings without any creative effort.
FIG. 1 is a schematic view of the overall structure of a multi-pole radiofrequency ablation catheter according to an embodiment of the present disclosure;
FIG. 2 is a partially exploded schematic view of the catheter assembly of the multi-pole radiofrequency ablation catheter shown in FIG. 1;
FIG. 3 is a partially cross-sectional view of the catheter assembly of the multi-pole radiofrequency ablation catheter shown in FIG. 1;
FIG. 4 is a partially exploded schematic view of the handle of the multi-pole radiofrequency ablation catheter shown in FIG. 1;
FIG. 5 is a schematic view of the overall structure of a multi-pole radiofrequency ablation catheter according to another embodiment of the present disclosure;
FIG. 6 is a partially exploded schematic view of the handle of the multi-pole radiofrequency ablation catheter shown in FIG. 5;
FIG. 7 is a schematic view of the overall structure of a multi-pole radiofrequency ablation catheter according to a further embodiment of the present disclosure;
FIG. 8 is a partially exploded schematic view of the handle of the multi-pole radiofrequency ablation catheter shown in FIG. 7;
FIG. 9 is a partially exploded schematic view of the catheter assembly of the multi-pole radiofrequency ablation catheter shown in FIG. 7;
FIG. 10 is a schematic diagram of an application scenario of a radiofrequency operation prompting method based on the multi-pole radiofrequency ablation catheter according to an embodiment of the present disclosure;
FIG. 11 is a flow chart of a radiofrequency operation prompting method based on the multi-pole radiofrequency ablation catheter according to an embodiment of the present disclosure;
FIG. 12 is a flow chart of a radiofrequency operation prompting method based on the multi-pole radiofrequency ablation catheter according to another embodiment of the present disclosure;
FIG. 13 is a schematic diagram of a radiofrequency operation prompting device according to an embodiment of the present disclosure;
FIG. 14 is a schematic diagram of a radiofrequency operation prompt system according to an embodiment of the present disclosure; and
FIG. 15 is a schematic diagram of a hardware arrangement of an electronic apparatus according to an embodiment of the present disclosure.

### DDESCRIPTION OF EMBODIMENTS

In order to make the purpose, technical solutions and advantages of the embodiments of the present disclosure apparent, the technical solutions of the embodiments of the present disclosure will be described clearly and completely in combination with the drawings accompanying the embodiments of the present disclosure. Obviously, the drawings described only show some embodiments of the present disclosure, but not all embodiments. Based on the embodiments of the present disclosure, all other embodiments obtained by those skilled in the art without creative efforts fall within the protection scope of the present disclosure.

In the description of the present disclosure, it should be noted that the terms such as "central", "longitudinal", "transversal", "upper", "lower", "front", "rear", "left", "right", "vertical", "horizontal", "top", "bottom", "inner", "outer", "axial", "radial" and "circumferential" for indicating the orientations or positional relationships refer to the orientation or positional relationship shown in the drawings, which are only for the convenience of describing the present disclosure and simplifying the description, but do not indicate or imply that the device or element referred to must have the specific orientations, be arranged and operated in the specific orientations, and therefore cannot be construed as limiting the scope of the present disclosure.

In the description of the present disclosure, "axial direction" refers to the direction parallel to the length direction of the multi-pole radiofrequency ablation catheter, "radial direction" refers to the direction perpendicular or approximately perpendicular to the axial direction, "circumferential direction" refers to the direction around the axial direction.

"Inner" / "outer" are relative concepts, which are used to indicate that a feature or an element where the feature is located is at least partly located radially inside / outside another feature or an element where the another feature is located.

"Proximal" and "distal" are relative concepts. "Proximal end" refers to the end close to the operator in the axial direction, which may be an end surface of a certain element, component or device close to the operator, or a part of a certain component, component or device close to the operator. "Distal end" refers to the end away from the operator in the axial direction, which may be an end surface of a certain element, component or device away from the operator, or a part of a certain component, component or device away from the operator.

In the present disclosure, unless otherwise clearly specified and defined, terms such as "install", "join", "connect" and "fix" should be interpreted in a broad sense. For example, "connection" may refer to a fixed connection or a detachable connection or being connected in one piece; a mechanical connection, an electrical connection, or a communication connection; a direct connection or indirect connection through an intermediate element; and an internal communication inside two elements or an interaction of two elements, unless otherwise defined. Those skilled in the art can understand the specific meanings of these terms herein in specific situations.

The technical solution of the embodiments of the present disclosure will be described in detail below. The same or similar concepts or processes in the following specific embodiments may not be repeated in some embodiments.

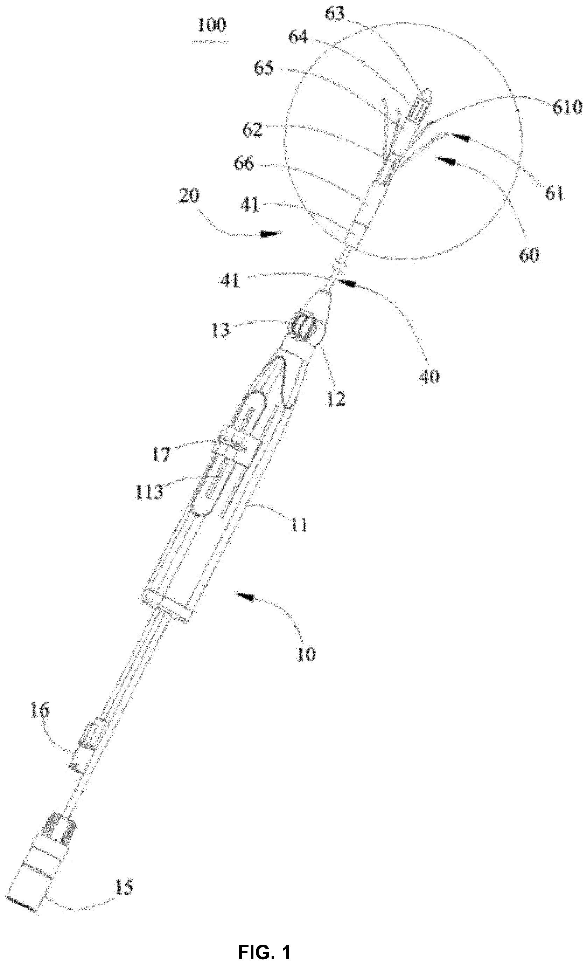

Referring to FIG. 1, a multi-pole radiofrequency ablation catheter 100 according to an embodiment of the present disclosure includes a handle 10 and a catheter assembly 20 connected to the handle 10. The catheter assembly 20 includes an outer catheter assembly 40 connected to the handle 10, and an inner catheter assembly 60 connected to the handle 10. The inner catheter assembly 60 can be driven by the handle 10 to rotate relative to the outer catheter assembly 40. Specifically, the proximal end of the outer catheter assembly 40 is connected to the distal end of the handle 10. The inner catheter assembly 60 includes a branched electrode assembly 61. The distal end of the branched electrode assembly 61 can protrude from the distal end of the outer catheter assembly 40, preferably by operating the handle 10. More preferably, the distal end of the branched electrode assembly 61 can expand outwardly and protrude from the distal end of the outer catheter assembly 40 in a claw shape. The branched electrode assembly 61 includes a plurality of side electrodes 610 arranged at intervals in the circumferential direction.

It can be seen from the above arrangement that the operator can rotate the branched electrode assembly 61 relative to the outer catheter assembly 40 by operating the handle 10. Therefore, when the catheter assembly 20 of the multi-pole radiofrequency ablation catheter 100 is introduced inside the lung under the guidance of B-scan ultrasonography or Computed Tomography, the blood vessel can pass through the gap between the adjacent side electrodes 610 of the branched electrode assembly 61, thereby avoiding piercing the blood vessel, and the outer catheter assembly 40 in this embodiment does not need to be rotated, with a convenient operation.

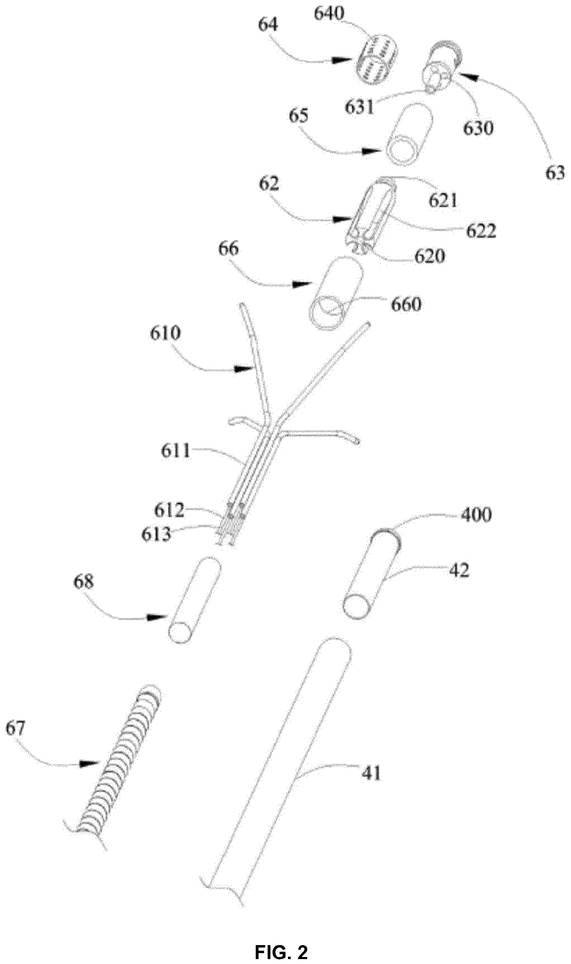

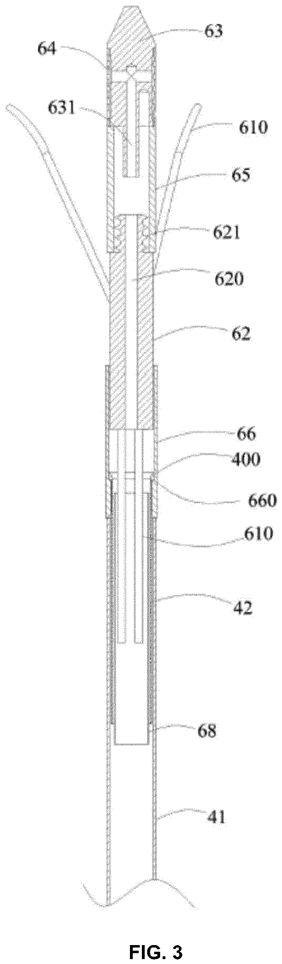

Referring to FIG. 1 to FIG. 3, in this embodiment, each side electrode 610 of the branched electrode assembly 61 includes a probe 611, and a sensor wire 612 and a guide wire 613 connected to the probe 611. After entering the human tissue, such as the lung, the deflection angles of the distal ends of the probes 611 of the side electrodes 610 relative to the central axis of the multi-pole radiofrequency ablation catheter 100 may be the same or different.

Optionally, the inner catheter assembly 60 further includes a support member 62 for supporting the branched electrode assembly 61. The support member 62 is generally cylindrical, and the side electrodes 610 of the branched electrode assembly 61 are distributed at intervals along the outer peripheral wall of the support member 62. In this embodiment, the support member 62 is arranged at the distal end of the outer catheter assembly 40, and can rotate together with the branched electrode assembly 61 relative to the outer catheter assembly 40. The branched electrode assembly 61 can slide axially relative to the support member 62.

In this embodiment, the inner catheter assembly 60 further includes a central electrode 63 fixedly connected to the distal end of the support member 62. Optionally, the center of the support member 62 is defined with a through hole 620 extending in the axial direction through the support member 62, for accommodating the sensor wire and guide wire of the central electrode 63 and a saline pipeline (not shown). The proximal end surface of the central electrode 63 is recessed to form a plurality of connection grooves 630. The sensor wire and the guide wire of the central electrode 63 are respectively connected to the respective connection grooves 630, and then enter the through hole 620 of the support member 62. A hollow liquid inlet column 631 protrudes from the center of the proximal end surface of the central electrode 63 for connecting with the saline pipeline extending from the through hole 620 of the support member 62. Preferably, the inner catheter assembly 60 further includes a wetting cover 64 surrounding the outer peripheral surface of the central electrode 63, and the wetting cover 64 is defined with a plurality of wetting holes 640 extending in the radial direction through the wetting cover 64. The wetting holes 640 are preferably distributed at regular intervals in the circumferential direction and the axial direction respectively. The saline in the saline pipeline enters the central electrode 63 through the liquid inlet column 631, and flows out from the wetting hole 640 through channels (not shown) in the central electrode 63, so as to diffuse in the human tissue, such as in the lung tissue.

In this embodiment, the inner catheter assembly 60 further includes a first connecting sleeve 65 for connecting the support member 62 and the central electrode 63. The first connecting sleeve 65 is in the shape of a hollow cylinder, preferably made of plastic. Preferably, the distal end of the first connecting sleeve 65 is melt-fitted with the wetting cover 64 and / or the central electrode 63. Preferably, a metal wire (not shown) is further connected between the first connecting sleeve 65 and the central electrode 63 to enhance the connection strength therebetween. One end of the metal wire can be fixed in the connection groove 630 in the proximal end surface of the central electrode 63, and the other end can be melted and connected with the first connecting sleeve 65. Preferably, the distal end of the support member 62 protrudes to form a hollow threaded post 621 aligned with the through hole 620, and the proximal end of the first connecting sleeve 65 is melt-fitted to the outer periphery of the threaded post 621 to strengthen the connection strength between the first connecting sleeve 65 and the support member 62, thereby preventing the first connecting sleeve 65 and components connected thereto such as the central electrode 63 from falling off.

In this embodiment, the proximal end surface of the support member 62 is opposite to the distal end surface of the outer catheter assembly 40, and the two can abut against each other so as to provide stable axial support for the support member 62 and prevent the support member 62 from moving proximally when rotating together with the branched electrode assembly 61. Alternatively, a certain gap may be reserved between the proximal end surface of the support member 62 and the distal end surface of the outer catheter assembly 40, so as to provide a certain degree of axial support for the support member 62 and also allow the support member 62 to rotate with the branched electrode assembly 61 flexibly.

In this embodiment, the inner catheter assembly 60 further includes a rotatable member 66 for connecting the support member 62 and the outer catheter assembly 40. The rotatable member 66 is generally tubular. The distal end of the rotatable member 66 is fixedly connected to the support member 62, for example, by interference fit or welding. The proximal end of the rotatable member 66 is rotatably connected to the outer catheter assembly 40.

Preferably, the outer wall of the support member 62 has a plurality of first grooves 622 distributed at intervals in the circumferential direction, and the length direction of the first groove 622 is consistent with the length direction of the support member 62 (in this embodiment, in the axial direction). Each side electrode 610 is slidably received in a corresponding first groove 622, and its distal end can slide into and out from the rotatable member 66. The first groove 622 helps the side electrode 610 to slide stably in the axial direction and prevent deflection. Preferably, the rotatable member 66 covers part of the length of the plurality of first grooves 622, so that the distal end sections of the plurality of first grooves 622 are still visible, which is convenient for judging the rotation direction of the support member 62, thereby judging the rotation direction of the branched electrode assembly 61 and thus the branched electrode assembly 61 avoiding the blood vessel.

Preferably, the inner wall of the rotatable member 66 is provided with a first protrusion 660 protruding radially inwardly, and the distal end of the outer catheter assembly 40 is provided with a second protrusion 400 protruding radially outwardly. The second protrusion 400 is rotatably supported on the distal end of the first protrusion 660, so that the rotatable member 66 can rotate together with the support member 62 and the branched electrode assembly 61 relative to the outer catheter assembly 40, and also the rotatable member 66 is prevented from moving distally when rotating together with the support member 62 and the branched electrode assembly 61, further, the rotatable member 66 and the components connected thereto, such as the supporting member 62, are prevented from falling off.

Preferably, the outer catheter assembly 40 includes an outer sheath 41 and a connecting tube 42 fixedly connected with each other. The proximal end of the outer sheath 41 is connected to the distal end of the handle 10, preferably fixedly connected with each other. The connecting tube 42 is accommodated in the distal end of the outer sheath 41 and fixedly connected with the outer sheath 41 by, for example, interference fit or welding. The second protrusion 400 is provided at the distal end of the connecting tube 42 connecting with the rotatable member 66.

More preferably, the distal end surface of the outer sheath 41 abuts against the proximal end surface of the rotatable member 66, thereby preventing the rotatable member 66 from moving proximally when rotating together with the support member 62 and the branched electrode assembly 61.

Optionally, in this embodiment, the inner catheter assembly 60 further includes a spring tube 67 accommodated in the outer catheter assembly 40, and the distal end of the spring tube 67 is directly or indirectly fixedly connected to the probes 611 of the branched electrode assembly 61. In this embodiment, the inner catheter assembly 60 further includes a second connecting sleeve 68 for connecting the spring tube 67 and the probes 611 of the branched electrode assembly 61. The proximal end of the second connecting sleeve 68 surrounds the outer periphery of the distal end of the spring tube 67 and is fixedly connected with the distal end of the spring tube 67. The distal end of the second connecting sleeve 68 surrounds the proximal end of the probes 611 of the branched electrode assembly 61 and is fixedly connected with the proximal ends of the probes 611 of the branched electrode assembly 61.

In this embodiment, the guide wires 613 and the sensor wires 612 of the branched electrode assembly 61 extend inside the spring tube 67 towards the proximal end (towards the handle 10). The saline pipeline and the sensor wire and guide wire of the central electrode 63 extend proximally from the through hole 620 of the support member 62 to the annular gap between the second connecting sleeve 68 and the connecting tube 42, and further extend proximally (towards handle 10) in the annular gap between the spring tube 67 and the outer sheath 41.

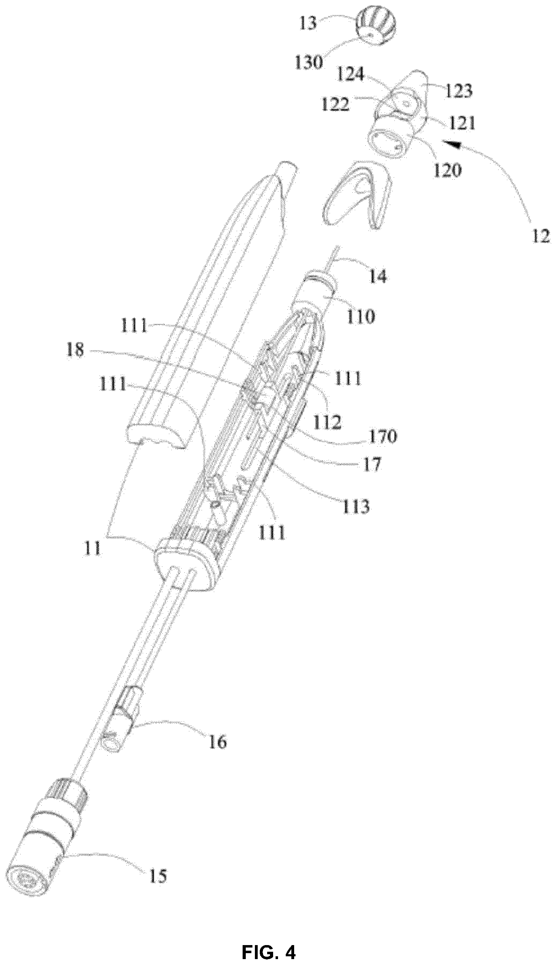

Referring to FIGS. 1 and 4, in this embodiment, the handle 10 includes a housing 11, an end cap 12 connected to the distal end of the housing 11, a rotatable ball 13 rotatably accommodated in the end cap 12, and a push-pull rod 14 connected with the rotatable ball 13 in an anti-rotational manner.

Specifically, in this embodiment, the housing 11 is generally in the shape of a hollow cylinder, and includes two half-housings that are snapped with each other. A cylindrical inner connector 110 is provided at the distal end of one of the half-housings. The end cap 12 includes a hollow cylindrical outer connector 120 that covers the outer periphery of the inner connector 110 and is connected with the inner connector 110, a plurality of arc-shaped connecting pieces 121 fixedly connected with the distal end of the outer connector 120 and defining a receiving chamber 122, and a tapered part 123 fixedly connected to the distal ends of the plurality of arc-shaped connecting pieces 121. An opening 124 is defined between adjacent arc-shaped connecting pieces 121. The outer sheath 41 is inserted into the tapered part 123 and is fixedly connected with the tapered part 123 in this embodiment. The rotatable ball 13 is accommodated in the receiving chamber 122, and the operator can rotate the rotatable ball 13 through the opening 124. The proximal end of the push-pull rod 14 is accommodated in the housing 11, and the distal end passes through the inner connector 110, the rotatable ball 13 in turn to the outer sheath 41, and is directly or indirectly connected to the spring tube 67. In this embodiment, the push-pull rod 14 is directly connected to the spring tube 67.

Preferably, the push-pull rod 14 is hollow. The guide wires 613 and sensor wires 612 of the branched electrode assembly 61 extend proximally in the aforementioned spring tube 67 into the push-pull rod 14, and further extend proximally to an electrode joint 15 fixed at the proximal end of the housing 11. More preferably, an accommodating space 111 is defined in the housing 11 for accommodating the saline pipeline and the sensor wire and guide wire of the central electrode 63 extending proximally from the annular gap between the spring tube 67 and the outer sheath 41 into the housing 11. Preferably, the accommodating space 111 is enclosed by the inner wall of the housing 11 and curved supports 112 (such as L-shaped supports) protruding from the inner wall. More preferably, two accommodating spaces 111 opposite in the radial direction are defined in the housing 11. The sensor wire and guide wire of the central electrode 63 are accommodated in the accommodating space 111 on one radial side and extend to and are connected with the electrode joint 15 fixed at the proximal end of the housing 11, and the saline pipeline is accommodated in the accommodating space 111 on the other radial side and extends to and is connected with a saline pipeline joint 16 fixed at the proximal end of the housing 11.

When the branched electrode assembly 61 needs to be rotated, the operator can rotate the rotatable ball 13. Since the rotatable ball 13 is connected with the push-pull rod 14 in an anti-rotational manner, the push-pull rod 14 will also rotate together with the rotatable ball 13. The rotation of the push-pull rod 14 drives the inner catheter assembly 60 (the spring tube 67, second connecting sleeve 68, branched electrode assembly 61, support member 62, rotatable member 66, first connecting sleeve 65, and the central electrode 63 and the wetting cover 64 connected with the first connecting sleeve 65) to rotate.

In this embodiment, by driving the push-pull rod 14 to move in the axial direction, the branched electrode assembly 61 can also be driven to move in the axial direction relative to the support member 62 and the rotatable member 66, thereby moving the distal ends of the probes 611 of the branched electrode assembly 61 out from or into the rotatable member 66.

Specifically, the handle 10 further includes a slidable assembly, which includes a slide button 17 slidably connected to the housing 11, a fixing block 18 rotatably connected to the slide button 17, and the push-pull rod 14. More specifically, the side wall of the housing 11 is defined with a slot 113 that passes through the housing 11 in the radial direction, and the slide button 17 is accommodated in the slot 113, with a part of the slide button 17 protruding from the outer surface of the housing 11 so that the operator can move the slide button 113, and the other part accommodated in the housing 11 and defining a receiving groove 170 that opens radially inwardly. The fixing block 18 is accommodated in the receiving groove 170 and preferably abuts against two axially opposite walls of the receiving groove 170. More preferably, the fixing block 18 is in the shape of a cylinder, so that the fixing block 18 can stably rotate relative to the slide button 17. The proximal end of the push-pull rod 14 passes through the slide button 17 and the fixing block 18. The push-pull rod 14 is fixedly connected with the fixing block 18, such as by interference fit. The push-pull rod 14 is movably connected with the slide button 17, for example by clearance fit. Thus, the push-pull rod 14 can rotate relative to the slide button 17, and the movement of the slide button 17 can be transmitted to the push-pull rod 14 through the fixing block 18, so that the push-pull rod 14 can drive the spring tube 67, the second connecting sleeve 68, and the branched electrode assembly 61 to move in the axial direction.

Preferably, a non-circular hole 130 is defined in the center of the rotatable ball 13, such as a square hole, an oval hole, etc., and the push-pull rod 14 passes through the rotatable ball 13 through the non-circular hole 130 and is connected with the wall of the non-circular hole 130. Thus, the push-pull rod 14 can move axially relative to the rotatable ball 13, and can also rotate together with the rotatable ball 13.

In practice of using the ablation catheter according to this embodiment, the distal end of the branched electrode assembly 61 is initially accommodated in the rotatable member 66. The distal end of catheter assembly 20, i.e., the central electrode 63, percutaneously punctures the target tumor. Then the rotatable ball 13 is rotated, and the rotation direction can be judged through the visible sections (distal sections) of the four first grooves 622 of the support member 62, so that the four first grooves 622 can avoid blood vessels. After rotating to a safe and appropriate posture, the central electrode 63 is inserted into the lesion site, and the slide button 17 is pushed to push the branched electrode assembly 61 out of the rotatable member 66 to obtain the conditions surrounding the central electrode 63, such as temperature and impedance, thereby judging the ablation progress. Since the first grooves 622 avoid the blood vessels, each side electrode 610 of the branched electrode assembly 61 pushed out from the rotatable member 66 will also avoid the blood vessels without piercing the blood vessels.

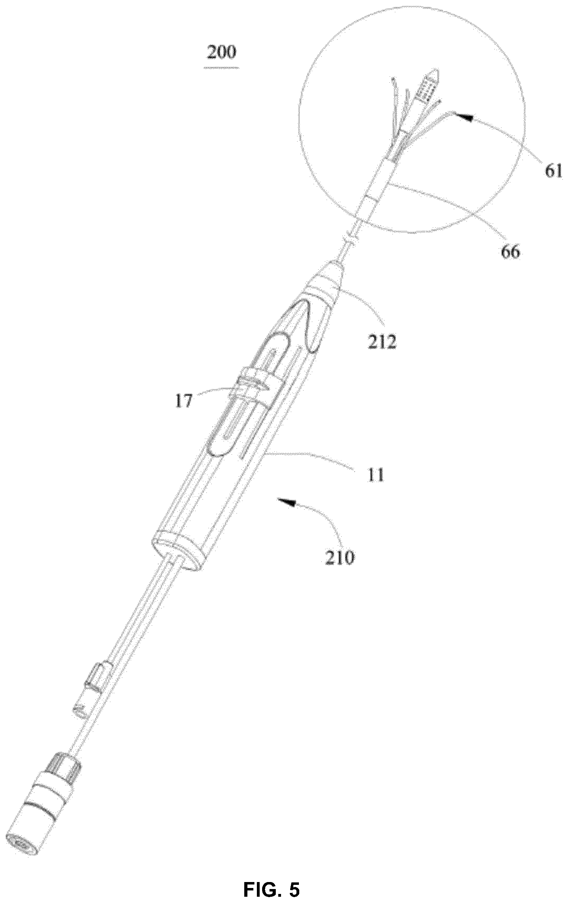

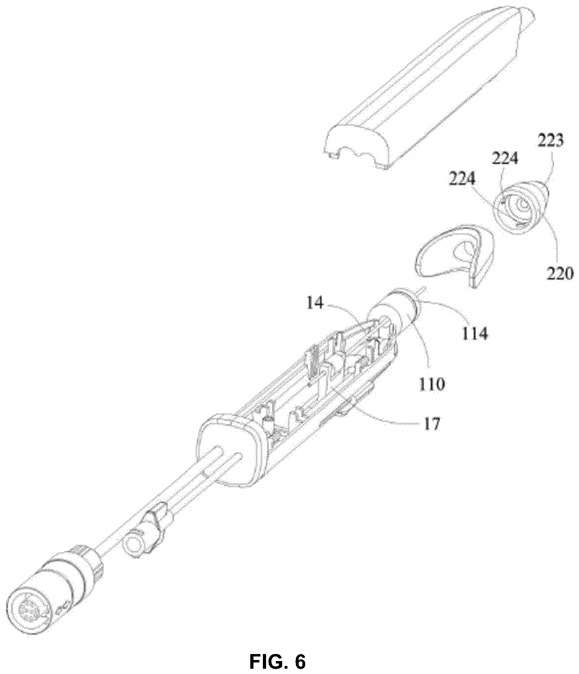

Referring to FIG. 5 and FIG. 6, a multi-pole radiofrequency ablation catheter 200 according to another embodiment of the present disclosure is similar to the multi-pole radiofrequency ablation catheter 100 according to the embodiment shown in FIG. 1 to FIG. 4, and the main difference therebetween lies in the mechanism for rotating the branched electrode assembly 61.

Specifically, the handle 210 of the multi-pole radiofrequency ablation catheter 200 in this embodiment no longer includes the end cap 12 and the rotatable ball 13 in the embodiment shown in FIGS. 1 to 4. Instead, in this embodiment, the end cap 212 of the handle 210 consists of a hollow cylindrical outer connector 220 that covers the outer periphery of the inner connector 110 and is rotatably connected with the inner connector 110, and a tapered part 223 fixedly connected with the distal end of the outer connector 220. The outer sheath 41 is inserted into the tapered part 223 and is fixedly connected with the tapered part 223 in this embodiment.

The inner connector 110 and the outer connector 220 are rotatably connected with each other using the following mechanism: the outer periphery of the inner connector 110 is recessed to form an annular slide groove 114, and the inner wall of the outer connector 220 protrudes to form one or more arc-shaped slide ring 224 or one annular slide ring 224 which is rotatably accommodated in the slide groove 114. It can be understood that in other embodiments, other mechanisms can be used to realize the rotatable connection between the end cap 212 and the handle 210.

Further, the push-pull rod 14 in this embodiment is no longer clearance-fitted with the slide button 17, instead, the push-pull rod 14 in this embodiment is fixedly connected with the slide button 17, such as by interference fit.

When in use, the operator can hold the end cap 212 with one hand, and turn the housing 11 of the handle 210 with the other hand. Since the push-pull rod 14 is fixedly connected with the slide button 17 in this embodiment, when the housing 11 is rotated, the push-pull rod 14 will also rotate together, thereby rotating the branched electrode assembly 61 through the spring tube 67 and the second connecting sleeve 68. When the branched electrode assembly 61 needs to be moved, the distal ends of the probes 611 of the branched electrode assembly 61 can be pushed out from or into the rotatable member 66 by pushing or pulling the slide button 17.

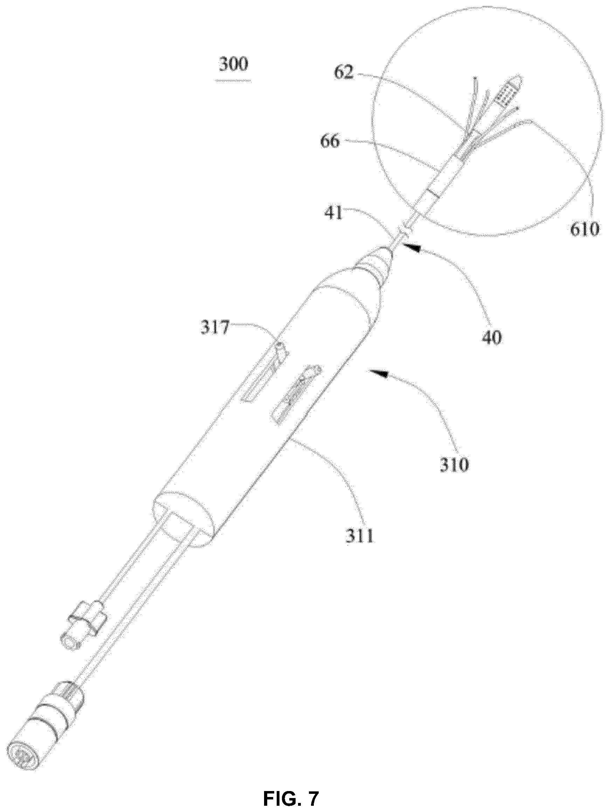

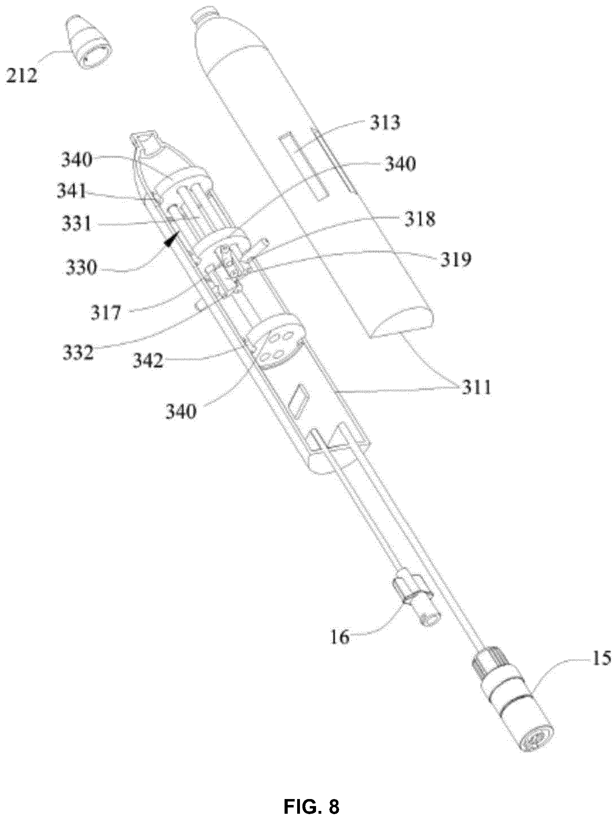

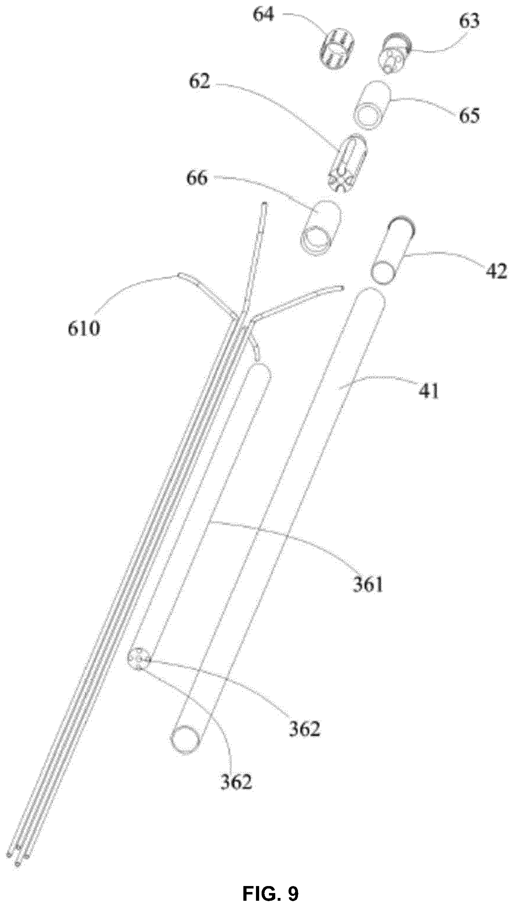

Referring to FIG. 7 to FIG. 9, a multi-pole radiofrequency ablation catheter 300 according to a further embodiment of the present disclosure is similar to the multi-pole radiofrequency ablation catheter 200 in the embodiment shown in FIG. 5 and FIG. 6, the main difference therebetween lies in the slidable assembly of the multi-pole radiofrequency ablation catheter 300 according to the present embodiment.

Specifically, in this embodiment, the slidable assembly of the multi-pole radiofrequency ablation catheter 300 includes a plurality of slidable elements 330 that can slide axially relative to the housing 311 separately. Each side electrode 610 of the branched electrode assembly 61 is fixedly connected with a corresponding slidable element 330 and can slide axially relative to the housing 311.

When using the multi-pole radiofrequency ablation catheter 300 according to this embodiment, the side electrodes 610 can be individually controlled to move in the axial direction. Therefore, the side electrodes 610 can be respectively moved to the ideal lesion areas by controlling the slidable elements 330 respectively to improve the positioning effect of the respective side electrodes 610, thereby improving the reliability of ablation monitoring results.

Optionally, the slidable element 330 includes a slidable rod 331 slidably arranged in the housing 311, and a slide button 317 fixedly connected with the slidable rod 331 and protruding from the outer surface of the housing 311. The side electrodes 610 are fixedly connected with the respective slidable rods 331. The slide buttons 317 are slidably connected with the housing 311.

Preferably, the handle 310 further includes at least one support seat 340 accommodated in the housing 311. In this embodiment, the support seat 340 is generally disc-shaped, and its outer peripheral wall abuts against and is fixedly connected to the inner peripheral wall of the housing 311. The slidable rod 331 passes through the at least one support base 340 in the axial direction and is slidably connected with the at least one support base 340. In this embodiment, the handle 310 includes three support seats 340 arranged at intervals in the axial direction. The slidable rod 331 is slidably connected with the middle support seat 340 and the distal support seat 340. The support seat 340 can not only provide a moving path for the slidable elements 330, so that the slidable elements 330 can move stably in the axial direction without deflection, but also support the housing 311 to prevent the housing 311 from deformation.

More preferably, the outer periphery of the support seat 340 has at least one second groove 341 extending in the axial direction through the support seat 340, and an accommodating space 342 is enclosed by the second groove 341 and the inner wall of the housing 311. In this embodiment, the outer periphery of each support seat 340 has two second grooves 341 that are radially opposite to each other. The saline pipeline extends proximally from the through hole 620 of the support member 62 through the connecting tube 42 and the outer sheath 41 of the outer catheter assembly 40 into the housing 311, and through the accommodating space 342 on one radial side enclosed by each support seat 340 and the housing 311 until being connected with the saline pipeline joint 16 fixed at the proximal end of the housing 311. The sensor wire and guide wire of the central electrode 63 extend proximally from the through hole 620 of the support member 62 through the connecting tube 42 and the outer sheath 41 of the outer catheter assembly 40 into the housing 311, and through the accommodating space 342 on the other radial side enclosed by each support seat 340 and the housing 311 until being connected with the electrode joint 15 fixed at the proximal end of the housing 311. The arrangement of the accommodating spaces 342 can prevent the wires of the multi-pole radiofrequency ablation catheter 300 from being entangled with each other.

Preferably, the slidable rod 331 is defined with a first receiving hole 332 extending in the axial direction through the slidable rod 331. The probe 611 of each side electrode 610 extends from the rotatable member 66 through the connecting tube 42 and the outer sheath 41 of the outer catheter assembly 40 in turn and into the first receiving hole 332 of a corresponding slidable rod 331 in the housing 311 and is fixedly connected with the slidable rod 331. The sensor wire 612 and the guide wire 613 of each side electrode 610 further extend proximally from the first receiving hole 332 through the proximal support base 340 until being connected with the electrode joint 15 fixed at the proximal end of the housing 311. The first receiving holes 332 and the proximal support seat 340 can effectively prevent the wires of the multi-pole radiofrequency ablation catheter 300 from being entangled with each other.

Preferably, the housing 311 has a plurality of slots 313 spaced from each other in the circumferential direction. Each slot 313 extends through the housing 311 in a radial direction, and the length direction of each slot 313 is consistent with the length direction of the housing 311 (that is, in the axial direction in this embodiment). The slide button 317 of each slidable element 330 protrudes from the outer surface of the housing 311 through a corresponding slot 313. The slide button 317 is limited by the slot 313, so that the slide button 317 can slide axially in the slot 313 stably, effectively preventing the slide button 317 from deflecting in the circumferential direction.

In this embodiment, the radially inner end of the slide button 317 of each slidable element 330 is vertically connected to the corresponding slidable rod 331. Preferably, the slide button 317 is arranged adjacent to the proximal end of the corresponding slidable rod 331. The sidewall of the slide button 317 adjacent to its radially inner end has a recessed avoidance groove 318, preferably, the avoidance groove 318 is located on the sidewall of the slide button adjacent to the proximal end of the slidable rod 331, so as to prevent the slidable element 330 form interference with surrounding components and / or wires etc. when moving the slidable element 330.

Preferably, the slidable element 330 further includes a wedge 319 disposed adjacent to the distal end of the slidable rod 331 and on the side of the slidable rod 331 opposite to the slide button 317. The slidable rod 331, the slide button 317, and the wedge 319 together form a generally T-shaped configuration. Specifically, the wedge 319 gradually tapers (reduces in depth) from the side wall of the slidable rod 331 toward the center of the slidable assembly (in this embodiment, i.e., the center of the four slidable elements 330, also the central axis of the handle 310). The wedge 319 not only helps the slidable element 330 to slide stably in the axial direction, but also helps to avoid interference between adjacent slidable elements 330.

Further preferably, the inner catheter assembly 360 of the multi-pole radiofrequency ablation catheter 300 according to this embodiment further includes a support rod 361 accommodated in the outer catheter assembly 40, and the support rod 361 has a plurality of second receiving holes 362 extending in the axial direction through the support rod 361. Preferably, the support rod 361 has a central second receiving hole 362, and a plurality of circumferential second receiving holes 362 surrounding the central second receiving hole 362. Each side electrode 610 passes through a corresponding circumferential second receiving hole 362. The saline pipeline and the sensor wire and guide wire of the central electrode 63 pass through the central second receiving hole 362. In other words, in this embodiment, the inner catheter assembly 360 no longer includes the spring tube 67 and the second connecting sleeve 68. The second receiving holes 362 can effectively prevent the wires of the multi-pole radiofrequency ablation catheter 300 from being entangled with each other.

When in use, the operator can hold the end cap 212 with one hand, and rotate the housing 311 of the handle 310 with the other hand. Since the branched electrode assembly 61 is fixedly connected with the slidable assembly of the handle 310, when the housing 311 is rotated, the branched electrode assembly 61 will also rotate together. When one or more side electrodes 610 of the branched electrode assembly 61 need to be moved, pushing or pulling the respective one or more slidable elements 330 can push or pull the distal end of the probe 611 of the respective side electrode 610 out from or into the rotatable member 66.

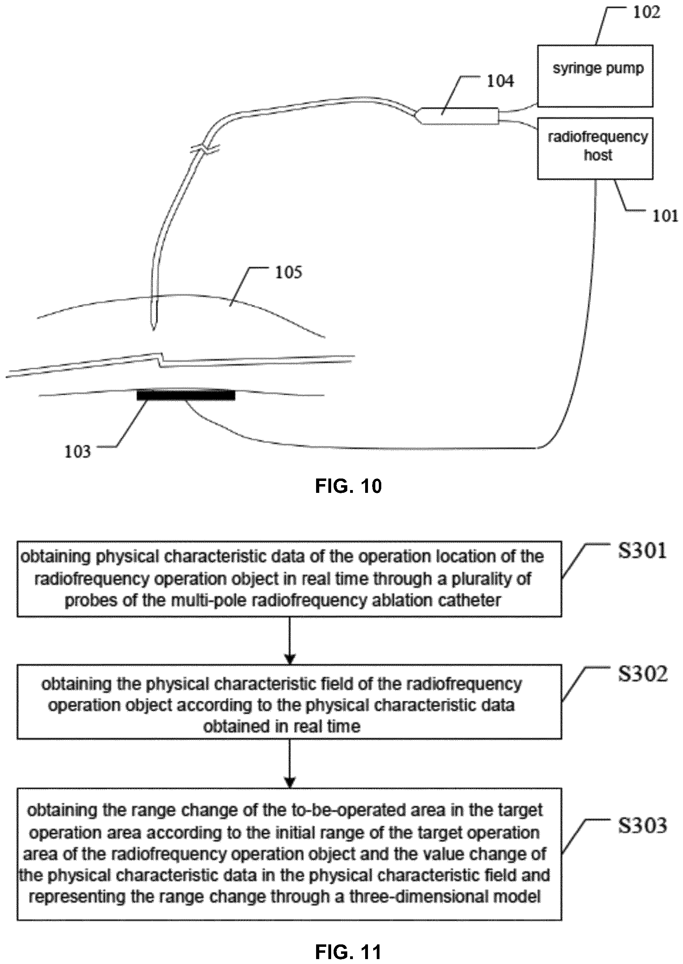

Referring to FIG. 10, a schematic diagram of an application scenario of a radiofrequency operation prompting method based on the multi-pole radiofrequency ablation catheter according to an embodiment of the present disclosure is shown. The radiofrequency operation prompting method based on the multi-pole radiofrequency ablation catheter according to the present disclosure can be realized by the radiofrequency host 10 shown in FIG. 10 or other computer devices that have data connections with the radiofrequency host 10.

As shown in FIG. 10, the radiofrequency host 101 is connected to a syringe pump 102, a neutral electrode 103 and a multi-pole radiofrequency ablation catheter 104 (the structure thereof can refer to FIGS. 1 to 9). The radiofrequency host 10 has a built-in display screen (not shown in the figure).

Specifically, before the operation, first, the top of the multi-pole radiofrequency ablation catheter 104 for generating and outputting radiofrequency energy and the extension tube (not shown in the figure) of the syringe pump 102 are inserted into the operation object 105 (such as an abnormal tissue mass). Then, the neutral electrode 103 is brought into contact with the surface of the operation object 105. The radiofrequency current flows through the multi-pole radiofrequency ablation catheter 104, the operation object 105 and the neutral electrode 103, thereby forming a loop.

When the operation is started, the radiofrequency host 101 controls the multi-pole radiofrequency ablation catheter 104 to output radiofrequency energy to the operation site by discharging, so as to perform radiofrequency operation on the operation site. At the same time, the syringe pump 102 performs perfusion operation on the operation object through the extension tube, and performs perfusion of physiological saline to the operation site to adjust the impedance and temperature of the operation site.

At the same time, the radiofrequency host 101 obtains the physical characteristic data of the operation location of the radiofrequency operation object in real time through the plurality of probes arranged at the top of the multi-pole radiofrequency ablation catheter 104. According to the physical characteristic data obtained in real time, the physical characteristic field of the radiofrequency operation object can be obtained. According to the initial range of the target operation area of the radiofrequency operation object and the value change of the physical characteristic data in the physical characteristic field, the range change of the to-be-operated area in the target operation area is obtained and presented through a three-dimensional model.

Referring to FIG. 11, a flow chart of a radiofrequency operation prompting method based on the multi-pole radiofrequency ablation catheter according to an embodiment of the present disclosure is shown. This method can be implemented by the radiofrequency host 101 shown in FIG. 10 or other computer terminals connected to it. For the convenience of description, the radiofrequency host 101 is used as the executing subject in the following embodiments. As shown in FIG. 11, the method specifically includes:
Step S301, obtaining physical characteristic data of the operation location of the radiofrequency operation object in real time through a plurality of probes of the multi-pole radiofrequency ablation catheter;
The specific structure of the multi-pole radiofrequency ablation catheter used in this embodiment can refer to FIGS. 1 to 9. Since the positions of the side electrodes of the multi-pole radiofrequency ablation catheter are adjustable, that is to say, the positions of the probes are adjustable, the flexibility of physical characteristic data detection can be improved.

Specifically, when the radiofrequency host controls the multi-pole radiofrequency ablation catheter to perform radiofrequency operation, the probes of the multi-pole radiofrequency ablation catheter surround the central electrode, as the central electrode punctures or touches the operation site of the radiofrequency operation object, the physical characteristic data at different locations of the operation site can be detected in real time. The physical characteristic data may specifically be temperature or impedance, or both.

The radiofrequency operation object refers to any object or target where radiofrequency ablation and other radiofrequency operations can be performed. For example, when the radiofrequency operation is radiofrequency ablation, the radiofrequency operation object can be a biological tissue, and the operation location can be abnormal tissue on the biological tissue.

Step S302: obtaining the physical characteristic field of the radiofrequency operation object according to the physical characteristic data obtained in real time;
Specifically, the physical characteristic data may include, but is not limited to, temperature data and impedance data, and accordingly, the physical characteristic field may include a temperature field and an impedance field.

The temperature field is a collection of temperature values at various points on the radiofrequency operation object, reflecting the distribution of temperature values in space and time, and can generally be represented as a function of spatial coordinates and time of the object. That is, t = f (x, y, z, τ ), among them, x, y, z are three rectangular spatial coordinates respectively, and τ is the time coordinate. In the prior art, there are many specific algorithms for the temperature field, which are not specifically limited in the present disclosure.

Similar to the temperature field, the impedance field is a collection of impedance values at various points on the radiofrequency operation object, a function of time and spatial coordinates, and reflects the distribution of impedance values in space and time.

Step S303, obtaining the range change of the to-be-operated area in the target operation area according to the initial range of the target operation area of the radiofrequency operation object and the value change of the physical characteristic data in the physical characteristic field and representing the range change through a three-dimensional model.

The target operation area is the execution area of the current radiofrequency operation on the radiofrequency operation object, and the initial range of the target operation area can be obtained by X-ray scanning or other fluoroscopy technologies.

The to-be-operated area is the area where the radiofrequency operation has not been executed or the effect has not reached the standard after the execution, and it is the area where the radiofrequency operation needs to be continued.

The value of the physical characteristic data at each point in the physical characteristic field changes at any time with the radiofrequency operation, and represents the phase of the radiofrequency operation. Specifically, when the value of the physical characteristic data reaches the preset threshold, it indicates that the radiofrequency operation is finished (that is, the expected effect has been achieved). When the value of the physical characteristic data is less than the preset threshold, it indicates that the radiofrequency operation is not finished (that is, the expected effect has not been achieved) or started. The area not finished or started is the to-be-operated area. That is, the range of the to-be-operated area can be determined according to the values of the physical characteristic data in the physical characteristic field. The range of the to-be-operated area also changes with the change of the measured values of the points in the physical characteristic field, and the change trend is that as the time of radiofrequency operation increases, the range of the to-be-operated area becomes smaller and smaller.

Through the presentation of the preset three-dimensional model software, the change of the range of the to-be-operated area in the target operation area is displayed on the display interface of the radiofrequency host, so as to intuitively provide the radiofrequency operator with the radiofrequency operation situation.

In the embodiment of the present disclosure, by using a plurality of probes of the multi-pole radiofrequency ablation catheter, a plurality of physical characteristic data at the radiofrequency operation location are obtained in real time, and the physical characteristic field of the radiofrequency operation object is obtained according to these data, and then according to the initial range of the target operation area of the radiofrequency operation object and the value change of the physical characteristic data in the physical characteristic field, the range change of the to-be-operated area in the target operation area can be obtained and represented through the three-dimensional model, realizing a visual prompt of range change of the to-be-operated area with more abundant, intuitive and vivid prompt information, improving the accuracy and intelligence of determining the to-be-operated area, thereby improving the effectiveness of the information prompt and thus the success rate and effect of radiofrequency operation.

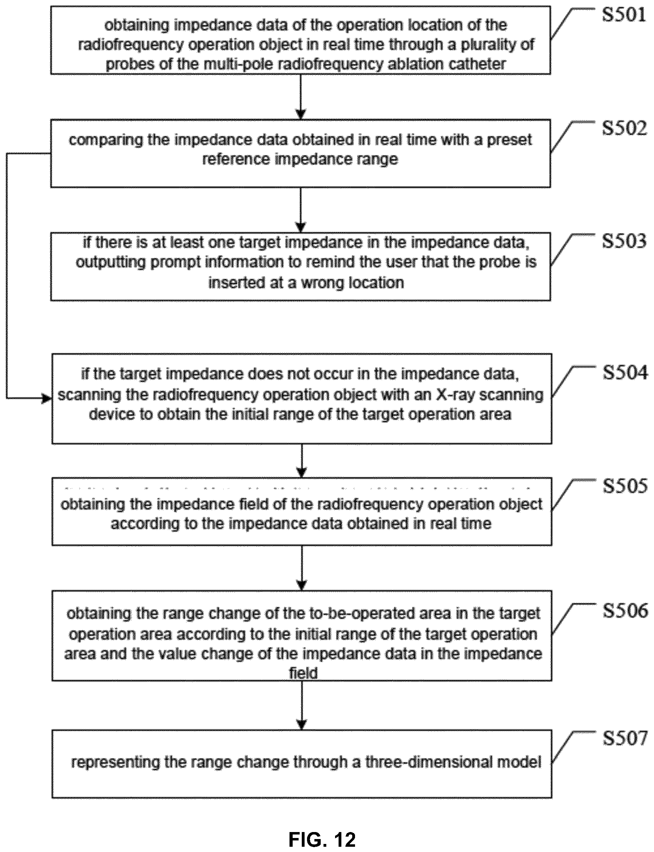

Referring to FIG. 12, a flow chart of a radiofrequency operation prompting method based on the multi-pole radiofrequency ablation catheter according to another embodiment of the present disclosure is shown. This method can be implemented by the radiofrequency host 101 shown in FIG. 10 or other computer terminals connected to it. For the convenience of description, the radiofrequency host 101 is used as the executing subject in the following embodiments. As shown in FIG. 12, the method specifically includes:
Step S501, obtaining impedance data of the operation location of the radiofrequency operation object in real time through a plurality of probes of the multi-pole radiofrequency ablation catheter;
Step S502, comparing the impedance data obtained in real time with a preset reference impedance range;
Step S503, if there is at least one target impedance in the impedance data, outputting prompt information to remind the user that the probe is inserted at a wrong location;
The specific structure of the multi-pole radiofrequency ablation catheter used in this embodiment can refer to FIGS. 1 to 9. It can be understood that the impedance value of normal biological tissue is different from the impedance value of abnormal biological tissue. A reference impedance database is configured in the radiofrequency host. The reference impedance database is used to store reference impedance ranges corresponding to biological tissues with different types of abnormalities (e.g., tumor, inflammation, cancer, etc.). By querying the reference impedance database, the reference impedance range corresponding to the abnormality type of the current radiofrequency operation object can be obtained. Optionally, the reference impedance database can be configured in the cloud.

The value of the target impedance doesn't fall within the reference impedance range. Comparing the impedance data obtained through the plurality of probes with the reference impedance range queried, if there is at least one target impedance in the obtained impedance data, it means that the probes have not completely covered the site that needs radiofrequency operation and the radiofrequency operation may fail to achieve the expected effect at the current location, then a preset prompt message will be output on the display screen to remind the user that the probe is inserted at a wrong location.

Further, the prompt information includes position information of the probe obtaining the target impedance, so that the user can determine the displacement direction of the probe according to the position information, with more intelligent information prompt.

Further, after outputting the prompt information, return to step S501 every preset time period until the target impedance does not occur in the impedance data; alternatively, in response to a control instruction triggered by the user by pressing a preset physical or virtual button, return to step S501.

By using the reference impedance range to prompt when the probe insertion location is wrong, the probe placing operation can be guided, with more intelligent information prompt, improving the speed of placing the probe, and shorting the time for the radiofrequency operation, improving the operation efficiency.

Step S504, if the target impedance does not occur in the impedance data, scanning the radiofrequency operation object with an X-ray scanning device to obtain the initial range of the target operation area;
Specifically, if there is no target impedance in the obtained impedance data, that is, the values of all the obtained impedance data fall into the reference impedance range, it means that the probes have completely covered the site that needs radiofrequency operation; then X-ray scanning device can be used to scan the target site of the radiofrequency operation object to obtain a three-dimensional image of the target site; and then image recognition is performed on the three-dimensional image to obtain the position coordinates of the probes in the three-dimensional image; and then the coverage of the probes can be determined according to the obtained position coordinates, and used as the initial range of the target operation area. The X-ray scanning device is, for example, a CT (Computed Tomography) machine.

Step S505, obtaining the impedance field of the radiofrequency operation object according to the impedance data obtained in real time;
Specifically, the impedance field is a collection of impedance values at various points on the radiofrequency operation object, a function of time and spatial coordinates, and reflects the distribution of impedance values in space and time.

Step S506, obtaining the range change of the to-be-operated area in the target operation area according to the initial range of the target operation area and the value change of the impedance data in the impedance field;
The to-be-operated area is the area where the radiofrequency operation has not been executed or the effect has not reached the standard after the execution, and it is the area where the radiofrequency operation needs to be continued. The value of the impedance data at each point in the impedance field changes at any time with the radiofrequency operation, and represents the phase of the radiofrequency operation. For example, when the value of the impedance data reaches the preset threshold, it indicates that the radiofrequency operation has achieved the expected effect. When the value of the impedance data is less than the preset threshold, it indicates that the radiofrequency operation has not achieved the expected effect or is not started. The area having not achieved the expected effect or not started is the to-be-operated area. That is, the range of the to-be-operated area can be determined according to the values of the impedance data in the impedance field. The range of the to-be-operated area also changes with the change of the measured values of the points in the impedance field, and the change trend is that as the time of radiofrequency operation increases, the range of the to-be-operated area becomes smaller and smaller.

Specifically, by comparing the value of the impedance data of each point in the impedance field with the preset threshold (that is, the preset impedance threshold), the boundary of the range of the to-be-operated area can be determined according to the points the values of the impedance data of which are greater than the preset threshold.

It can be understood that, by fitting the points the values of the impedance data of which are greater than the preset threshold together by using a preset fitting algorithm, the range of the area in the target operation area where the expected effect has been achieved can be obtained. By comparing the initial range of the target operation area and the range of the area where the expected effect has been achieved, the range of the to-be-operated area and the boundary thereof can be obtained. The preset fitting algorithm may be, but not limited to, for example, least square method or Matlab curve fitting algorithm, which is not specifically limited in the present disclosure.

Further, when the value of the impedance data of the operation location is greater than the preset threshold, the range of the radiation area of the operation location can be determined according to the values of the impedance data of the operation location, the detection angle of the probe corresponding to the operation location and the preset radiation distance, and according to the range of the radiation area, the boundary of the to-be-operated area can be determined.

It can be understood that since the radiofrequency energy output by the central electrode of the multi-pole radiofrequency ablation catheter radiates into the biological tissue along a specific direction, the impedance change has a radiation range.

The detection angle of the probe is the angle at which the probe used to detect the impedance of a certain operation location punctures or contacts the operation location. The radiation direction can be determined from the detection angle. According to the values of the impedance data of the operation location, the radiation direction, and the preset radiation distance, the range of the radiation area of the operation location, that is, the depth of the boundary of the range of the area where the expected effect has been achieved can be determined.

Step S507, representing the range change through a three-dimensional model.

Specifically, a 3D model of the range change of the to-be-operated area can be constructed according to the three-dimensional image of the target operation area obtained by using the X-ray scanning device and the range change of the to-be-operated area in the target operation area, using the surface-rendering based Marching Cubes algorithm on or the volume-rendering based Ray-casting algorithm, Shear-warp, Frequency Domain and Splatting algorithm and other algorithms, and represented on the preset display interface.

Among them, the Marching Cubes algorithm uses a series of two-dimensional slice data as a three-dimensional data field, and constructs the surface mesh of the three-dimensional model by extracting the isosurface of the three-dimensional data, and thereby constructing the three-dimensional model. The volume-rendering based algorithm directly converts the discrete data in the three-dimensional space into the final stereo image without generating intermediate geometric primitives, and the principle thereof is to assign an opacity to each voxel and involve the impact of each voxel on light transmission, emission and reflection.

Optionally, in another embodiment of the present disclosure, a plurality of probes are used to obtain temperature data of the operation location of the radiofrequency operation object in real time, and the method includes the following steps:
Step S701, obtaining temperature data of the operation location of the radiofrequency operation object in real time through the plurality of probes;
Step S702, obtaining the initial range of the target operation area by scanning the radiofrequency operation object with an X-ray scanning device;
Step S703, obtaining the temperature field of the radiofrequency operation object according to the temperature data obtained in real time;
Step S704, obtaining the range change of the to-be-operated area in the target operation area according to the initial range of the target operation area and the value change of the temperature data in the temperature field;
Step S705, representing the range change through a three-dimensional model.

The above step S701 to step S705 are similar to step S501 and step S504 to step S507, the detail thereof can refer to the relevant description of the above step S501 and step S504 to step S507, which will not be repeated here.

Optionally, in another embodiment of the present disclosure, a plurality of probes are used to obtain the temperature data and the impedance data of the operation location of the radiofrequency operation object in real time, and the method includes the following steps:
Step S801, obtaining the impedance data and the temperature data of the operation location of the radiofrequency operation object in real time through the plurality of probes;
Step S802, comparing the impedance data obtained in real time with a preset reference impedance range;
Step S803, if there is at least one target impedance in the impedance data, outputting prompt information to remind the user that the probe is inserted at a wrong location;
Step S804, if the target impedance does not occur in the impedance data, scanning the radiofrequency operation object with an X-ray scanning device to obtain the initial range of the target operation area;
Step S805, obtaining the impedance field and the temperature field of the radiofrequency operation object according to the impedance data and the temperature data obtained in real time;
Step S806, obtaining the range change of the to-be-operated area in the target operation area according to the initial range of the target operation area, the value change of the impedance data in the impedance field and the value change of the temperature data in the temperature field;
Step S807, representing the range change through a three-dimensional model.

The above step S801 to step S805 and step S807 are similar to the above step S501 to step S505 and step S507, the detail thereof can refer to the relevant description of the above step S501 to step S505 and step S507, which will not be repeated here.

The difference is that step S805 specifically includes: comparing the values of the temperature data of the points in the temperature field with a preset temperature threshold, and determining the first boundary of the to-be-operated area according to the points the values of the temperature data of which are greater than the preset temperature threshold; after a preset time period, comparing the values of the impedance data of the points in the impedance field with the preset impedance threshold, and modifying the first boundary of the to-be-operated area according to the points the values of the impedance data of which are greater than the preset impedance threshold to obtain a second boundary, and determining the second boundary as the boundary of the to-be-operated area.

Modifying the first boundary of the to-be-operated area according to the points the values of the impedance data of which are greater than the preset impedance threshold to obtain a second boundary means that for the same point, when the value of the impedance data is greater than the preset impedance threshold, while the value of the temperature data is not greater than the preset temperature threshold, the impedance data shall prevail, and the position corresponding to this point shall be determined as the position where the expected effect has been achieved.

Using the temperature field to first determine the first boundary, and then using the impedance field to modify the first boundary can achieve a complementary effect, obtaining a more accurate final boundary.

Optionally, the method further includes: determining the unit variation of the to-be-operated area according to the range change of the to-be-operated area at regular intervals; determining the remaining time for finishing the current radiofrequency operation according to the unit variation and the current volume of the to-be-operated area, and outputting prompt information through the display interface.

Specifically, the regular interval is determined depending on the preset time, and every preset time period, the unit variation of the to-be-operated area, for example, the volume of the to-be-operated area reducing per second, can be obtained by dividing the amount of the range change of the to-be-operated area by the executed time of the radiofrequency operation. Then, the remaining time for finishing the current radiofrequency operation can be obtained by multiplying the current volume of the to-be-operated area by the unit variation.

The current volume of the to-be-operated area can be obtained by the initial volume of the target operation area minus the unit variation of the to-be-operated area. The initial volume of the target operation area can be determined according to the three-dimensional image of the target operation area scanned by the X-ray scanning device in step S504.

By prompting the remaining time for finishing the current radiofrequency operation, the user can clearly know the progress of the radiofrequency operation, further improving the intelligence of the information prompt.

In the embodiment of the present disclosure, by using a plurality of probes of the multi-pole radiofrequency ablation catheter, a plurality of physical characteristic data at the radiofrequency operation location are obtained in real time, and the physical characteristic field of the radiofrequency operation object is obtained according to these data, and then according to the initial range of the target operation area of the radiofrequency operation object and the value change of the physical characteristic data in the physical characteristic field, the range change of the to-be-operated area in the target operation area can be obtained and represented through the three-dimensional model, realizing a visual prompt of range change of the to-be-operated area with more abundant, intuitive and vivid prompt information, improving the accuracy and intelligence of determining the to-be-operated area, thereby improving the effectiveness of the information prompt and thus the success rate and effect of radiofrequency operation.

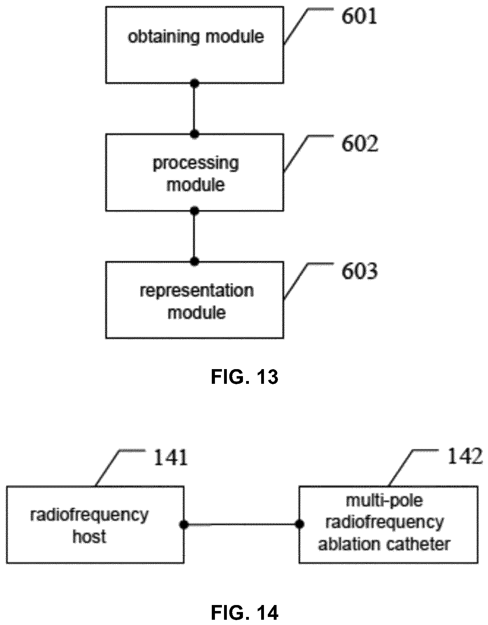

Referring to FIG. 13, a schematic diagram of a radiofrequency operation prompting device according to an embodiment of the present disclosure is shown. For ease of description, only parts related to the embodiment of the present disclosure are shown. The device may be a computer terminal, or a software module configured in the computer terminal. As shown in FIG. 13, the device includes an obtaining module 601, a processing module 602 and a representation module 603.
The obtaining module 601 is configured to obtain the physical characteristic data of the operation location of the radiofrequency operation object in real time through a plurality of probes of the multi-pole radiofrequency ablation catheter;
The processing module 602 is configured to obtain the physical characteristic field of the radiofrequency operation object according to the physical characteristic data obtained in real time;
The processing module 602 is further configured to obtain the range change of the to-be-operated area in the target operation area according to the initial range of the target operation area of the radiofrequency operation object and the value change of the physical characteristic data in the physical characteristic field;
The representation module 603 is configured to represent the range change through a three-dimensional model.

Further, the processing module 602 is also configured to compare the values of the physical characteristic data of the points in the physical characteristic field with a preset threshold; and determine the boundary of the range of the to-be-operated area according to the points the values of the physical characteristic data of which are greater than the preset threshold.

The processing module 602 is further configured to, when the value of the physical characteristic data of the operation location is greater than the preset threshold, determine the range of the radiation area of the operation location according to the value of the physical characteristic data of the operation location, the detection angle of the probe corresponding to the operation location and the preset radiation distance, in which the value of the physical characteristic data in the radiation area is greater than the preset threshold; and determine the boundary of the to-be-operated area according to the range of the radiation area.

Further, the physical characteristic data includes temperature data and / or impedance data, and the physical characteristic field includes a temperature field and / or an impedance field.

Further, when the physical characteristic data includes temperature data and impedance data, and the physical characteristic field includes the temperature field and the impedance field, the processing module 602 is further configured to compare the values of the temperature data of the points in the temperature field with the preset temperature threshold, and determine the first boundary of the to-be-operated area according to the points the values of the temperature data of which are greater than the preset temperature threshold;

After a preset time period, compare the values of the impedance data of the points in the impedance field with the preset impedance threshold, and modify first boundary of the to-be-operated area according to the points the values of the impedance data of which are greater than the preset impedance threshold to obtain a second boundary which is determined as the boundary of the to-be-operated area.

Further, the processing module 602 is also configured to determine the unit variation of the to-be-operated area according to the range change of the to-be-operated area at regular intervals; and determine the remaining time for finishing the current radiofrequency operation according to the unit variation and the current volume of the to-be-operated area;
The representation module 603 is further configured to output the remaining time as prompt information through a display interface.

Further, when the physical characteristic data includes impedance data, the processing module 602 is further configured to, compare the impedance data obtained in real time with the preset reference impedance range, and, if there is at least one target impedance in the impedance data, trigger the representation module 603 to output prompt information to prompt the user that the probe is inserted at a wrong location, and the value of the target impedance doesn't fall within the reference impedance range; and
If the target impedance does not occur in the impedance data, trigger the step of obtaining the physical characteristic field of the radiofrequency operation object according to the physical characteristic data obtained in real time.

Further, the processing module 602 is further configured to obtain the initial range of the target operation area by using an X-ray scanning device to scan the radiofrequency operation object.

The specific process for realizing the respective functions of the above-mentioned modules can refer to the related description in the embodiments shown in FIG. 11 and FIG. 12, which will not be repeated here.

In the embodiment of the present disclosure, by using a plurality of probes of the multi-pole radiofrequency ablation catheter, a plurality of physical characteristic data at the radiofrequency operation location are obtained in real time, and the physical characteristic field of the radiofrequency operation object is obtained according to these data, and then according to the initial range of the target operation area of the radiofrequency operation object and the value change of the physical characteristic data in the physical characteristic field, the range change of the to-be-operated area in the target operation area can be obtained and represented through the three-dimensional model, realizing a visual prompt of range change of the to-be-operated area with more abundant, intuitive and vivid prompt information, improving the accuracy and intelligence of determining the to-be-operated area, thereby improving the effectiveness of the information prompt and thus the success rate and effect of radiofrequency operation.

Referring to FIG. 14, a schematic diagram of a radiofrequency operation prompt system according to an embodiment of the present disclosure is shown. As shown in FIG. 14, the system includes a radiofrequency host 141 and a multi-pole radiofrequency ablation catheter 142.

The radiofrequency host 141 is configured to execute the steps of the radiofrequency operation prompting method based on the multi-pole radiofrequency ablation catheter described in the above-mentioned embodiments, for example, shown in FIG. 11 and FIG. 12 .

The multi-pole radiofrequency ablation catheter 142 can refer to the embodiments shown in FIGS. 1 to 9, for example, including a handle with a proximal end and a distal end, an outer catheter assembly with a proximal end and a distal end, and an inner catheter assembly with a proximal end and a distal end. The proximal end of the outer catheter assembly is connected to the distal end of the handle, and the proximal end of the inner catheter assembly is connected to the distal end of the handle. The inner catheter assembly includes a branched electrode assembly that can be driven by the handle to rotate relative to the outer catheter assembly. The branched electrode assembly includes a plurality of side electrodes arranged at intervals in the circumferential direction, and the side electrodes include the probes.

The specific process for realizing the functions of the radiofrequency host 141 can refer to the relevant descriptions in the aforementioned embodiments. The specific structure of the multi-pole radiofrequency ablation catheter 142 can refer to the relevant descriptions in the aforementioned embodiments shown in FIGS. 1 to 9, which will not be repeated here.

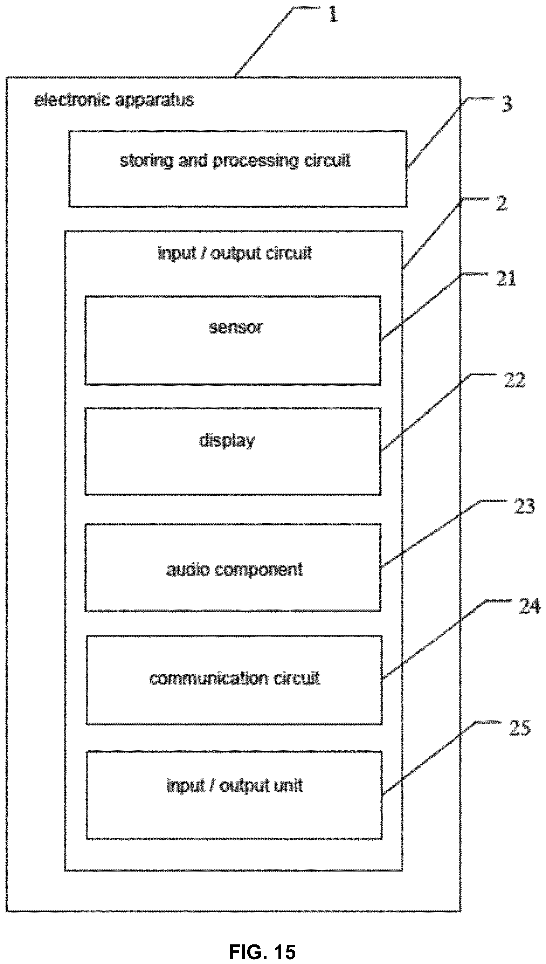

Referring to FIG. 15, a schematic diagram of a hardware arrangement of an electronic apparatus according to an embodiment of the present disclosure is disclosed.

For example, the electronic apparatus may be any of various types of computer system devices that are non-removable or removable or portable and that perform wireless or wired communication. Specifically, the electronic apparatus may be a desktop computer, a server, a mobile phone or a smart phone (for example, a phone based on iPhone TM, or based on Android TM), a portable game device (for example, Nintendo DS TM, PlayStation Portable TM, Gameboy Advance TM, iPhone TM), a laptop, a PDA, a portable Internet device, a portable medical device, a smart camera, a music player and a data storage device, other handheld devices and, for example, watch, earphones, pendant, headset, etc.. The electronic apparatus may be other wearable devices (e.g., such as electronic glasses, electronic cloth, electronic bracelet, electronic necklace, and other head-mounted devices (HMDs)).

As shown in FIG. 15, the electronic apparatus 1 may include a control circuit, which may include a storing and processing circuit 3. The storing and processing circuit 3 may include a memory, such as hard disk drive memory, non-transitory or non-volatile memory (such as flash memory or other electronically programmable limited-erasable memory for forming solid-state drive, etc.), volatile memory (such as static or dynamic random access memory, etc.), and the like, which is not limited in this embodiment of the present disclosure. The processing circuit in the storing and processing circuit 3 may be configured to control the operation of the electronic apparatus 1. The processing circuit may be embodied based on one or more microprocessors, microcontrollers, digital signal processors, baseband processors, power management units, audio codec chips, application specific integrated circuits, display driver integrated circuits, and the like.

The storing and processing circuit 3 can be configured to run software in the electronic apparatus 1, such as Internet browsing applications, Voice over Internet Protocol (VOIP) telephone call applications, email applications, media player applications, operating system functions, etc. The software can be configured to perform control operations, such as image acquisition based on camera, ambient light measurement based on ambient light sensor, proximity sensor measurement based on proximity sensor, information display based on status indicators such as LEDs, touch event detection based on touch sensor, information display on a plurality of (e.g. layered) displayers, operations related to wireless communication, operations related to audio signal collection and generation, control operations related to data collection and process of button press event, and other functions in the electronic apparatus 1, which are not limited in the present embodiment of the present disclosure.

Further, the memory stores executable program codes, and the processor coupled to the memory is configured to invoke the executable program codes stored in the memory to execute the radiofrequency operation prompting method based on the multi-pole radiofrequency ablation catheter as described in the above-mentioned embodiments. The processor is also electrically coupled with the multi-pole radiofrequency ablation catheter in the embodiments shown in FIGS. 1 to 9 through data lines.

The executable program codes include various modules in the radiofrequency operation prompting device described in the above embodiment shown in FIG. 13, for example, the obtaining module 601, processing module 602 and representation module 603. The specific process for realizing the respective functions of the above-mentioned modules can refer to the relevant description in FIG. 13, which will not be repeated here.

The electronic apparatus 1 may further include an input / output circuit 2. The input / output circuit 2 can be configured to enable the electronic apparatus 1 to realize data input and output, that is, allow the electronic apparatus 1 to receive data from external devices and also allow the electronic apparatus 1 to output data from the electronic apparatus 1 to external devices. The input / output circuit 2 may further include a sensor 21. The sensor 21 may be an ambient light sensor, a proximity sensor based on light and capacitance, a touch sensor (for example, a light and/ or capacitance based touch sensor which can be provided as a part of the touch panel or used independently), acceleration sensor, or other sensors, etc.

The input / output circuit 2 may further include one or more displays, such as display 22. The display 22 may include one or more of liquid crystal display, organic light emitting diode display, electronic ink display, plasma display, and displays using other display technologies. The display 22 may include a touch sensor array, that is, the display 22 may be a touch panel. The touch sensor may be a capacitive touch sensor formed from an array of transparent touch sensor electrodes such as indium tin oxide (ITO) electrodes, or may be a touch sensor formed using other touch technologies, such as acoustic touch, pressure sensitive touch, resistive touch, optical touch, etc., which are not limited in the present embodiment of the disclosure.

The electronic apparatus 1 may further include an audio component 23. The audio component 23 may be configured to provide audio input and output for the electronic apparatus 1. The audio component 23 in the electronic apparatus 1 may include speaker, microphone, buzzer, tone generator and other components for generating and detecting sounds.

A communication circuit 24 can be configured to provide the electronic apparatus 1 with the ability to communicate with external devices. The communication circuit 24 may include an analog and digital input / output interface circuit, and a wireless communication circuit based on radio frequency signals and / or optical signals. The wireless communication circuit in the communication circuit 24 may include a radio frequency transceiver circuit, a power amplifier circuit, a low noise amplifier, a switch, a filter, and an antenna. For example, the wireless communication circuit in the communication circuit 24 may include a circuit for supporting Near Field Communication (NFC) by transmitting and receiving near field coupled electromagnetic signals. For example, the communication circuit 24 may include a near field communication antenna and a near field communication transceiver. The communication circuit 24 may further include a cellular telephone transceiver and antennas, a wireless local area network transceiver circuit and antennas, and the like.

The electronic apparatus 1 may further include a battery, a power management circuit and other input / output unit 25. The input / output unit 25 may include a button, joystick, click wheel, scroll wheel, touch pad, keypad, keyboard, camera, light emitting diode and other status indicators, and the like.

The user can input instructions through the input / output circuit 2 to control the operation of the electronic apparatus 1, and can receive status information and other outputs from the electronic apparatus 1 through the output data of the input / output circuit 2.

Further, the embodiment of the present disclosure provides a non-transitory computer-readable storage medium, which can be configured in the servers of the above-mentioned embodiments, and a computer program is stored in the non-transitory computer-readable storage medium, and when the program is executed by the processor, the radiofrequency operation prompting method based on the multi-pole radiofrequency ablation catheter described in the above-mentioned embodiments is implemented.

The above embodiments are described from different aspects, and aspects of some embodiments that are not described in detail can refer to relevant aspects of other embodiments.

Those skilled in the art would know that the exemplary modules / units and algorithm steps described in the embodiments disclosed herein can be implemented by electronic hardware, or a combination of computer software and electronic hardware. Depending on the specific application and design of the technical solutions, these functions can be realized by hardware or software. Those skilled in the art may use different methods to realize the described functions for each specific application, which should not be regarded as exceeding the scope of the present disclosure.

In the embodiments provided in the present disclosure, it should be understood that the disclosed devices / terminals and methods may be implemented in other manners. For example, the devices / terminals described above are only illustrative. For example, the division of the modules or units is only a division from the perspective of logical function, and in practice, other division manners can be used. For example, a plurality of modules or components can be combined or integrated into another system, or some features may be omitted or don't work. In another aspect, the mutual coupling or direct coupling or communication connection shown or discussed may be indirect coupling or communication connection between devices or units via some interfaces, in electrical, mechanical or other forms.

The units described as separate components may or may not be physically separated, and the components displayed as units may or may not be physical units, that is, they may be located in one place, or may be distributed on a plurality of network units. Part or all of the units can be used according to practical needs to achieve the purposes of the solutions of the embodiments.

In addition, the functional units in each embodiment of the present disclosure may be integrated into one processing unit, or may present separately physically, or two or more units may be integrated into one unit. The above-mentioned integrated units can be implemented in the form of hardware or in the form of software units.

If the integrated units are implemented in the form of software units and sold or used as independent products, they can be stored in a computer-readable storage medium. In view of this, all or part of the processes implemented in the methods of the above embodiments in the present disclosure may also be implemented by instructing related hardware through computer programs. The computer programs can be stored in a computer-readable storage medium, and when the computer program is executed by a processor, the steps of the above-mentioned various method embodiments can be implemented. The computer program includes computer program codes, and the computer program codes may be in the form of source code, object code, executable file or some intermediate form. Computer-readable media may include any entity or device, recording media, U disk, removable hard disk, magnetic disk, optical disk, computer memory, read-only memory (ROM), random access Memory (RAM), electrical carrier signal, telecommunication signal and software distribution medium, etc., that is capable of carrying computer program codes. It should be noted that the content contained in the computer readable media may be appropriately increased or decreased according to the requirements of legislation and patent practice in the jurisdiction. For example, in some jurisdictions, according to legislation and patent practice, the computer readable media does not include electrical carrier signal and telecommunication signal.

The above embodiments are only intended to explain the technical solutions of the present disclosure, rather than for limitation. Although the present disclosure has been described in detail with reference to the foregoing embodiments, those skilled in the art should understand that it is still possible to modify the technical solutions described in the foregoing embodiments, or make equivalent replacements for some or all of the technical features, and these modifications or replacements do not make the essence of the corresponding technical solutions deviate from the scope of the technical solutions of the various embodiments of the present disclosure, and should be included in the scope of the present invention.

## Claims

1. A radiofrequency operation prompting method based on a multi-pole radiofrequency ablation catheter, applied to a computer terminal, wherein the method comprises steps of:
obtaining physical characteristic data of a operation location of a radiofrequency operation object in real time through a plurality of probes of the multi-pole radiofrequency ablation catheter;
obtaining a physical characteristic field of the radiofrequency operation object according to the physical characteristic data obtained in real time; and
obtaining a range change of an to-be-operated area in a target operation area according to an initial range of the target operation area of the radiofrequency operation object and a value change of the physical characteristic data in the physical characteristic field, and representing the range change through a three-dimensional model;
wherein the multi-pole radiofrequency ablation catheter comprises a handle with a proximal end and a distal end, an outer catheter assembly with a proximal end and a distal end, and an inner catheter assembly with a proximal end and a distal end; and wherein
the proximal end of the outer catheter assembly is connected with the distal end of the handle;
the proximal end of the inner catheter assembly is connected with the distal end of the handle; and
the inner catheter assembly comprises a branched electrode assembly that is capable of being driven by the handle to rotate relative to the outer catheter assembly and comprises a plurality of side electrodes arranged at intervals in a circumferential direction, and the side electrodes include the probes.

2. The method of claim 1, wherein the step of obtaining the range change of the to-be-operated area in the target operation area according to the initial range of the target operation area of the radiofrequency operation object and the value change of the physical characteristic data in the physical characteristic field comprises:
comparing values of the physical characteristic data of points in the physical characteristic field with a preset threshold; and
determining a boundary of the to-be-operated area according to the points the values of the physical characteristic data of which are greater than the preset threshold.

3. The method of claim 1 or 2, wherein the step of obtaining the range change of the to-be-operated area in the target operation area according to the initial range of the target operation area of the radiofrequency operation object and the value change of the physical characteristic data in the physical characteristic field further comprises:
when the values of the physical characteristic data of the operation location are greater than the preset threshold, determining a range of a radiation area of the operation location according to the values of the physical characteristic data of the operation location, a detection angle of the probe corresponding to the operation location and a preset radiation distance, wherein the values of the physical characteristic data in the radiation area are greater than the preset threshold; and
determining a boundary of the to-be-operated area according to the range of the radiation area.

4. The method of claim 3, wherein the physical characteristic data comprises a temperature data and / or an impedance data, and the physical characteristic field comprises a temperature field and / or an impedance field.

5. The method of claim 4, wherein when the physical characteristic data comprises the temperature data and the impedance data, and the physical characteristic field comprises the temperature field and the impedance field, the step of obtaining the range change of the to-be-operated area in the target operation area according to the initial range of the target operation area of the radiofrequency operation object and the value change of the physical characteristic data in the physical characteristic field comprises:
comparing the values of the temperature data of the points in the temperature field with a preset temperature threshold, and determining a first boundary of the to-be-operated area according to the points the values of the temperature data of which are greater than the preset temperature threshold; and
after a preset time period, comparing the values of the impedance data of the points in the impedance field with a preset impedance threshold, and modifying the first boundary of the to-be-operated area according to the points the values of the impedance data of which are greater than the preset impedance threshold to obtain a second boundary, and determining the second boundary as the boundary of the to-be-operated area.

6. The method of claim 5, further comprising:
determining a unit variation of the to-be-operated area according to the range change of the to-be-operated area at regular intervals; and
determining a remaining time for finishing a current radiofrequency operation according to the unit variation and a current volume of the to-be-operated area, and outputting the remaining time as a prompt information through a display interface.

7. The method of claim 1, wherein when the physical characteristic data comprises the impedance data, before the step of obtaining the physical characteristic field of the radiofrequency operation object according to the physical characteristic data obtained in real time, the method further comprises:
comparing the impedance data obtained in real time with a preset reference impedance range;
if there is at least one target impedance in the impedance data, outputting a prompt information to prompt a user that the probe is inserted at a wrong location, and the value of the target impedance doesn't fall within the reference impedance range; and
if the target impedance does not occur in the impedance data, the step of obtaining the physical characteristic field of the radiofrequency operation object according to the physical characteristic data obtained in real time is executed.

8. The method of any one of claims 1 to 6, wherein before the step of obtaining the physical characteristic field of the radiofrequency operation object according to the physical characteristic data obtained in real time, the method further comprises:
obtaining the initial range of the target operation area by scanning the radiofrequency operation object with an X-ray scanning device.

9. The method of claim 1, wherein the inner catheter assembly further comprises a support member for supporting the branched electrode assembly, and the plurality of side electrodes of the branched electrode assembly are arranged at intervals in the circumferential direction of the support member, and wherein the support member is rotatably connected with the outer catheter assembly.

10. The method of claim 9, wherein the support member has a proximal end and a distal end, a proximal end surface of the support member is opposed to a distal end surface of the outer catheter assembly, and the inner catheter assembly further comprises a rotatable member for connecting the outer catheter assembly and the support member, the rotatable member has a proximal end and a distal end, the distal end of the rotatable member is fixedly connected with the support member, and the proximal end of the rotatable member is rotatably connected with the outer catheter assembly.

11. The method of claim 10, wherein an outer wall of the support member has a plurality of grooves distributed at intervals in the circumferential direction, a length direction of the groove is consistent with a length direction of the support member, the rotatable member covers part of the length of the plurality of grooves, and each of the side electrodes is slidably accommodated in the respective groove and capable of sliding into or out from the rotatable member.

12. The method of claim 10, wherein the rotatable member is tubular, an inner wall of the rotatable member is provided with a first protrusion, and the distal end of the outer catheter assembly is provided with a second protrusion, and wherein the first protrusion is rotatably engaged with the second protrusion.

13. The method of claim 12, wherein the outer catheter assembly comprises an outer sheath having a proximal end and a distal end, and a connecting tube fixedly connected with the distal end of the outer sheath, the proximal end of the outer sheath is connected with the distal end of the handle, the connecting tube has a proximal end and a distal end, and the second protrusion is provided on the distal end of the connecting tube.

14. The method of claim 13, wherein the proximal end of the connecting tube is accommodated in the distal end of the outer sheath, and a distal end surface of the outer sheath abuts against a proximal end surface of the rotatable member.

15. A method of any one of claims 1 to 14, wherein the handle comprises a housing having a proximal end and a distal end, and an end cap rotatably connected with the distal end of the housing, and wherein the proximal end of the outer catheter assembly is connected with the end cap, and the housing is rotatable relative to the end cap so as to drive the inner catheter assembly to rotate relative to the outer catheter assembly.

16. The method of claim 15, wherein the handle further comprises a slidable assembly slidably connected with the housing, the slidable assembly is connected with the branched electrode assembly and capable of driving the branched electrode assembly to slide in the axial direction, and the housing is rotatable relative to the end cap so as to drive the slidable assembly to drive the branched electrode assembly to rotate relative to the outer catheter assembly.

17. The method of claim 16, wherein the slidable assembly comprises a plurality of slidable elements slidably connected with the housing, each of the slidable elements is at least partially disposed within the housing, and a proximal end of each of the side electrodes extends into the housing and is fixedly connected with the respective slidable element.

18. The method of claim 15, wherein the slidable assembly comprises a push-pull rod at least partially disposed within the housing and slidably connected with the housing, and the inner catheter assembly further comprises a spring tube received in the outer catheter assembly, the spring tube has a proximal end and a distal end, the proximal end of the spring tube is fixedly connected with the push-pull rod, and the distal end of the spring tube is fixedly connected with the branched electrode assembly.

19. The method of claim 18, wherein the slidable assembly further comprises a slide button slidably connected with the housing, a part of the slide button protrudes from an outer surface of the housing, and the other part of the slide button is accommodated in the housing and fixedly connected with the push-pull rod.

20. The method of any one of claims 1 to 14, wherein the handle comprises a housing having a proximal end and a distal end, an end cap connected with the distal end of the housing, a rotatable ball received in the end cap, and a push-pull rod connected with the rotatable ball in an anti-rotational manner, the proximal end of the outer catheter assembly is connected with the end cap, the push-pull rod is connected with the inner catheter assembly, and the rotatable ball is rotatable relative to the end cap so as to drive the push-pull rod to drive the inner catheter assembly to rotate relative to the outer catheter assembly.

21. The method of claim 20, wherein the handle further comprises a slide button slidably connected with the housing, and a fixing block rotatably connected with the slide button, the push-pull rod is fixedly connected with the fixing block and slidable axially relative to the rotatable ball, and the slide button is axially slidable relative to the housing so as to drive the push-pull rod to slide axially through the fixing block.

22. The method of claim 21, wherein a non-circular hole is defined in the rotatable ball, and the push-pull rod passes through the rotatable ball through the non-circular hole and connects with a wall of the non-circular hole.

23. An electronic apparatus, comprising:
a memory and a processor;
the memory stores executable program codes;
the processor is coupled with a multi-pole radiofrequency ablation catheter and the memory and configured to invoke the executable program codes stored in the memory to execute the steps of the radiofrequency operation prompting method based on the multi-pole radiofrequency ablation catheter of any one of claims 1 to 8.

24. A radiofrequency operation prompt system, comprising a radiofrequency host and a multi-pole radiofrequency ablation catheter; wherein
the radiofrequency host is configured to execute the steps of the radiofrequency operation prompting method based on the multi-pole radiofrequency ablation catheter of any one of claims 1 to 8;
wherein the multi-pole radiofrequency ablation catheter comprises a handle with a proximal end and a distal end, an outer catheter assembly with a proximal end and a distal end, and an inner catheter assembly with a proximal end and a distal end; and wherein
the proximal end of the outer catheter assembly is connected with the distal end of the handle;
the proximal end of the inner catheter assembly is connected with the distal end of the handle; and
the inner catheter assembly comprises a branched electrode assembly that is capable of being driven by the handle to rotate relative to the outer catheter assembly and comprises a plurality of side electrodes arranged at intervals in a circumferential direction, and the side electrodes include the probes.

25. A non-transitory computer-readable storage medium, in which a computer program is stored, wherein when the computer program is executed by a processor, the radiofrequency operation prompting method based on the multi-pole radiofrequency ablation catheter of any one of claims 1 to 8 is implemented.
